(19) Europäisches Patentamt
European Patent Office
Office européen des brevets

(11) Publication number: **0 088 994 B1**

(12) # EUROPEAN PATENT SPECIFICATION

(45) Date of publication of patent specification: **19.06.91**

(51) Int. Cl.[5]: **C12N 15/13**, C12P 21/02, A61K 39/395, G01N 33/531

(21) Application number: **83102288.4**

(22) Date of filing: **09.03.83**

(54) **Hybrid DNA, binding composition prepared thereby and processes therefor.**

(30) Priority: **15.03.82 US 358414**

(43) Date of publication of application:
**21.09.83 Bulletin 83/38**

(45) Publication of the grant of the patent:
**19.06.91 Bulletin 91/25**

(84) Designated Contracting States:
**AT BE CH DE FR GB IT LI NL SE**

(56) References cited:
**EP-A- 0 036 776**
**EP-A- 0 043 980**

**Chemical Abstracts, vol. 96, no. 9, March 1, 1982, page 128, abstract 63597c, Columbus, Ohio, US G. Matthyssens et al.: "Structure and arrangement of human heavy chain variable region genes" & Immune Syst. 1981, 1, 132-8**

(73) Proprietor: **SCHERING CORPORATION**
**2000 Galloping Hill Road**
**Kenilworth New Jersey 07033(US)**

(72) Inventor: **Moore, Kevin W.**
**3880 Fleetwood Drive**
**San Bruno California 94066(US)**
Inventor: **Zaffaroni, Alejandro**
**168 Isabella Avenue**
**Atherton California 94025(US)**

(74) Representative: **Ritter, Stephen David et al**
**Mathys & Squire 10 Fleet Street**
**London EC4Y 1AY(GB)**

**EP 0 088 994 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical Abstracts, vol. 93, no. 5, August 4, 1980, page 677, abstract 43563y, Columbus, Ohio, US M. Steinmetz et al.: "Cloning of V region fragments from mouse liver DNA and localization of repetitive DNA sequences in the vicinity of immunoglobulin gene segments"

Chemical Abstracts, vol. 89, no. 19, page 403, November 6, 1978, abstract 161353v, Columbus, Ohio, US J.G. Seidman et al.: "Multiple related immunoglobulin varibaleregion gens identified by cloning and sequence analysis"

Chemical Abstracts, vol. 87, no. 19, November 7, 1977, page 360, abstract 149414s, Columbus, Ohio, US S. Tonegawa et al.: "Cloning of an immunoglobulin variable region gene from mouse embryo"

Nucleic Acids Research, vol. 8, no. 18, 1980, D. Goeddel et al.: "Synthesis of human fibroblast interferon by E. coli", pages 4057-4074

## Description

The mammalian immunological system is unique in its broad ability to produce protein compounds, known as immunoglobulins, having extremely high specificity for a particular molecular structure. That is, these proteins have a conformation which is specifically able to complement a particular structure, so that binding occurs with high affinity. In this manner, the mammalian immune system is able to respond to invasions of foreign molecules, particularly proteins in surface membranes of microorganisms, and to toxins, resulting in detoxification or destruction of the invader, without adverse effects on the host.

The primarily immunoglobulin of the defensive mechanism is gamma-globulin (IgG). This immunoglobulin, which is a glycoprotein of about 150,000 daltons, has four chains, two heavy chains and two light chains. Each chain has a variable region and a constant region. The variable regions are concerned with the binding specificity of the immunoglobulin, while the constant regions have a number of other functions which do not directly relate to the antibody affinity.

In many situations it would be desirable to have binding molecules which, though substantially smaller than the immunoglobulins, still provide the specificity and affinity which the immunoglobulins afford. Smaller molecules can provide for shorter residence times in a mammalian host. In addition, where the immunoglobulin has to be bound to another molecule, it will be frequently desirable to minimize the size of the final product. Also there are many economies in being able to produce a smaller molecule which fulfills the function of a larger molecule.

There are situations where it is desirable to be able to have a large number of molecules compactly held together. By having smaller molecules, a greater number can be brought together into a smaller space. Furthermore, where such a binding molecule can be prepared by hybrid DNA technology, one has the opportunity to bind the binding portion of the molecule to a wide variety of other polypeptides, so that one can have the binding molecule covalently bonded at one or both ends to a polypeptide chain.

Where immunoglobulins are used in in vivo diagnosis or therapy, antisera from an allogenic host or from a monoclonal antibody may be immunogenic. Furthermore, when conjugates of other molecules to the antibody are employed, the resulting conjugate may become immunogenic and elicit host antibodies against the constant region of the immunoglobulin or against any other part of the molecule.

It is therefore important that methods be developed which permit the preparation of homogeneous compositions that comprise such binding molecules and have high specificity for a particular antigen or ligand but avoid the shortcomings of complete immunoglobulins and also afford the many advantages of lower molecular weight.

Discussions concerning variable regions of heavy and light chains of immunoglobulins may be found in Sharon and Givol, Biochem. (1976) 15:1591-1594; Rosenblatt and Haber, Biochem. (1978) 17:3877-3882; and Early and Hood, Genetic "Engineering (1981) 3:157-188. Synthesis of part of a mouse immunoglobulin light chain in a bacterial clone is described by Amster et al., Nucleic Acids Res. (1980) 8:2055-2065. Various references cited throughout the specification concern particular methodologies and compositions.

EP-A-0043980 is concerned with a process for the preparation of a polypeptide comprising the amino acid sequence of mature human leukocyte interferon.

An article entitled "Multiple related immunoglobin variable-regions identified by cloning and sequence analysis". Proc. Natl. Acad. Sci. U.S.A., Vol. 75, pages 3881-3885 describes the cloning of a DNA sequence coding for the variable region of an immunoglobin molecule.

Neither document discloses or suggests the specific binding compositions to which the present invention relates.

According to the present invention there is provided a specific binding composition comprising two polypeptide chains of an immunoglobulin having binding specificity to a predetermined ligand, characterized in that said two polypeptide chains

(1) have the amino acid sequence of at least a portion of the variable region of an immunoglobulin, but substantially lack the constant region,

(2) associate to form a complex having a high affinity and specificity for said predetermined ligand, and

(3) are the light chain of from about 95 to 115 amino acids and the heavy chain of from about 110 to 125 amino acids, wherein said heavy chain includes the D-region.

The polypeptide chains can be obtained by cultivation of genetically engineered microorganisms. Employing hybrid DNA technology, cDNA is tailored to remove nucleotides extraneous to the variable regions of the light and heavy chains. The resulting tailored ds cDNA is inserted into an appropriate expression vector which is then introduced into a host for transcription and translation. The resulting truncated light and heavy chains define at least a major portion of the variable regions and associate to form a complex capable of specifically binding with high affinity to an antigen or ligand at a haptenic side thereof.

The binding constant will generally be greater than $10^5$, more usually greater than $10^6$, and preferably greater than $10^8$.

Generally the polypeptide chains of the variable regions of the light and heavy chains will be employed together for binding to the ligand. However, it may exceptionally be possible to use a single chain if this chain has sufficient binding affinity to the ligand in question.

The two polypeptide chains which, individually or together, provide the compositions of this invention will form a receptor site, analogous to the binding site of an immunoglobulin. The composition will be referred to as an rFv with the individual chains referred to as L-rFv or K-rFv. The L- and H- designations will normally mean light and heavy respectively; sometimes the two chains may be the same and derived from either the light or heavy chain sequences. The polypeptide chains of the rFv will generally have fewer than 125 amino acids, more usually fewer than about 120 amino acids, while normally having more than 60 amino acids, usually more than about 95 amino acids, more usually more than about 100 amino acids. The H-rFv will be from about 110 to 125 amino acids while the L-rFv will be from about 95 to 115 amino acids.

The amino acid compositions will vary widely, depending upon the particular idiotype involved. Usually there will be at least two cysteines separated by from about 60 to 75 amino acids and joined by a disulfide link (forming cystine) to define a domain. The two chains will normally be substantial copies of idiotypes of the variable regions of the light and heavy chains of immunoglobulins, but in some situations it may be sufficient to have combinations of either the light or the heavy variable region chains.

It will often be desirable to have one or both of the rFv chains labeled, e.g. with a radiosotope , fluorescer, or toxin, or bound to an inert physiologically acceptable support, such as synthetic organic polymers, polysaccharides, naturally occurring proteins, or other non-immunogenic substances.

It may sometimes be desirable to provide for covalent crosslinking of the two chains, e.g. by providing for cysteine residues at the carboxyl termini. The chains will normally be prepared free of the constant regions; the J region may be present in part or in whole, or absent. The D region will be included in the transcript of the K-rFv.

Generally only a relatively small percent of the total amino acids will vary from idiotype to idiotype in the rFv. Therefore, there will be areas providing a relatively constant framework and areas that will vary, namely, the hypervariable regions.

The C-terminus region of the rFv will have a greater variety of sequences than the N-terminus and, based on the present invention, can be further modified to permit variation from the naturally occurring heavy and light chains. A synthetic oligonucleotide can be used to introduce mutations encoding different amino acids in a hypervariable region.

The preparation of the rFv by means of hybrid DNA technology will first be described in general terms and then in greater detail.

To provide a homogeneous rFv having high binding affinity, the mammalian immune system can be used as starting point. The messenger$^{RNA}$ from a hybridoma cell or other monoclonal antibody-producing cell is isolated and used to prepare a cDNA transcript encoding the light and/or heavy chains of the immunoglobulin. Based on the flanking sequences upstream and downstream, at the start (maybe including leader region) and finish of the DNA encoding the variable region, short DNA sequences (oligonucleotides) at least partially complementary to those sequences are employed for primer repair or in vitro mutagenesis to remove extraneous flanking regions and to introduce translational control signals. The in vitro mutagenesis employs an oligonucleotide, which heteroduplexes with one of the strands of the cDNA, in combination with Klenow fragment of DNA polymerase I. Primer repair requires a homoduplexing oligonucleotide in combination with the same enzyme. The process is carried out twice (conveniently once with the coding strand and once with the non-coding strand) to provide ds cDNA coding for the variable region with translational regulatory signals at predetermined sites. This ds cDNA is inserted into an appropriate vector, e.g. plasmid, to provide a hybrid vector capable of self-replication and having the proper regulatory signals for replication, selection and expression.

This hybrid vector is then introduced into an appropriate host to express the variable regions of the heavy or light polypeptide chains of the rFv and the polypeptides are isolated. The variable regions of the heavy and light polypeptide components of the rFv are then associated in an appropriate medium to form the rFv.

Since the idiotypes vary, the sequence of steps of the present invention permits one to handle a wide variety of coding sequences for variable regions. Also, the ds cDNA and vector can be tailored to optimize the regulatory signals which are employed, particularly the promoter. The ribosome binding site and variable-region initiation codon may be properly spaced to optimize expression. of the variable-region polypeptide.

The hybrid vectors containing the variable region coding sequence in the proper orientation are used to

transform the appropriate host for expression. The resulting transformants are selected by virtue of the markers present in the vector and then cloned and expanded. The polypeptide produced by the transformants may be isolated by separation of the cells and isolation of the supernatant into which such polypeptides are secreted; or, if the polypeptides are not secreted, the transformant cells are isolated and lysed, and the polypeptide is recovered. Fractions containing enhanced amounts of the variable region polypeptide may be obtained by various conventional techniques, such as gel electrophoresis, fractional preciptation, affinity chromatography, high pressure liquid chromatography, or the like. In any event, the original lysate, or supernatant, or the concentrated fractions therefrom, may be screened for the presence of the variable-region polypeptides by immunoassay.

A heavy or light chain that is secreted may be isolated as follows. Polyclonal antisera to monoclonal immunoglobulin can be prepared by immunizing an appropriate vertebrate with the whole monoclonal antibody, so as to produce antiserum which recognizes the determinant sites of the heavy and light chains. Antibodies recognizing the whole immunoglobulin or only the light or heavy chain may be substantially separated and purified from other antibodies in the antiserum. By binding to and eluting from affinity columns containing whole immunoglobulin, or only the heavy or light chains, covalently linked to an appropriate support, the antibodies for the whole immunoglobulin, or for the heavy or light chain respectively, become bound to the column. The column is denatured, and the purified antibodies are removed and then conjugated to an appropriate support to provide an affinity column to purify the heavy or light chains of the rFv.

Where the light or heavy chain is not secreted, the transformed microorganisms containing the appropriate ds cDNA for either light or heavy chains are grown in liquid cultures and cleared lysates are prepared, e.g. by treatment of the microorganisms with lysozyme, rupture of the cell membrane, centrifuging and collecting the clear liquids These lysates are then passed over an immunosorbent affinity column prepared as described above, employing the specific polyclonal antisera. The bound variable regions are eluted from the column with an appropriate denaturing solvent. The eluates from each of the heavy and light chain isolations are pooled and then treated to renature the polypeptides to form L-rFv and H-rFv respectively. For renaturation, the pooled eluates may be dialyzed against appropriate aqueous buffered solutions. The mixture is then further purified by passing it over the appropriate ligand-affinity column and the bound molecules eluted with an appropriate denaturing solvent. The variable regions are then renatured as previously described to provide a solution of rFvs which may be used for a variety of purposes.

In accordance with the present invention, molecules are provided which are polypeptide duplexes of the variable region of light and heavy chains of immunoglobulins, retaining the specificity of the immunoglobulins. By lacking the constant regions, the rFvs are less immunogenic and may, therefore, be prepared from sources xenogenic to a host to which they are to be administered, Furthermore, the rFvs are a homogeneous mixture, rather than a heterogeneous mixture, (The heterogeneous mixtures, which will contain chains of varying lengths, could be obtained by other techniques, such as enzyme and acid treatment.) The homogeneity of the compositions of the present invention allows for uniform modification and accurate determination of therapeutic levels. In addition, there is no contamination with chains from whole immunoglobulins, which, if inadequately digested, would retain immunogenic portions or uncover new immunogenic sites. Finally, large amounts of the desired rFvs may be prepared in high yield and high purity.

The present invention provides furthermore appropriate transformed expression vectors or plasmids carrying a ds DNA sequence coding for said rFvs; transformed hosts (such as bacteria, e.g. E. coli, or yeasts) carrying such expression vectors; methods for preparing such transformed expression vectors or plasmids; and methods for preparing said rFvs by cultivating such transformed hosts.

The transformed expression vector or plasmid according to the invention carries a ds DNA sequence that codes for a variable region of a light or heavy chain of an immunoglobulin specific for a predetermined ligand but lacks nucleotides coding for aminoacid residues superfluous to said variable region and is equipped for initiation and termination codons at the 5'- and 3'-termini respectively of said DNA sequence.

The ligand may be for example an enzyme or a surface protein. The ds DNA sequence may code for example for 2 variable region of a chain having about 95 to 125 amino acids, in particular for a variable region of a light chain having 95 to 115 amino acids or for a variable region of a heavy chain having about 110 to 125 amino acids, especially for at least the D region of the heavy chain.

The invention further provides a method for preparing a transformed expression vector or plasmid which carries a ds DNA sequence that codes for a variable region of a light or heavy chain of an immunoglobulin specific for a predetermined ligand but lacks nucleotides coding for amino acid residues superfluous to said variable region and is equipped for initiation and termination codons at the 5'-and 3'-termini respectively of

said DNA sequence;
said method comprising:
preparing ds cDNA encoding at least one of said light or heavy chains from an mRNA coding for said chain;
removing nucleotide sequences from said ds cDNA superfluous to said variable region and providing for initiation and termination codons at the 5'- and 3'-termini respectively of the DNA sequence to provide tailored ds cDNA encoding said variable region;
and inserting said tailored ds cDNA into an expression vector for expression of said ds cDNA.

The initiation and termination codons can be provided by in vitro mutagenesis. If desired, the method may include the additional step, prior to said inserting, of replacing at least one nucleotide in said ds cDNA to change a codon to encode for a different amino acid.

A particularly preferred embodiment of this method comprises the following steps a) to f):

a) preparing ds cDNA coding for a light or heavy chain of an immunoglobulin, each chain being composed of a constant region and a variable region, said variable regions having about 95 to 125 amino acids, by the steps of isolating mRNA that codes for said chain, reverse-transcribing said mRNA to produce ss cDNA, synthesizing a strand complementary to said ss cDNA by means of DNA polymerase to produce ds cDNA having a coding strand coding for said light or heavy chain, wherein said coding strands include DNA sequences that code for leader sequence, variable region and constant region of said immunoglobulin in the 5'-3' direction of said coding strand, and cloning said ds cDNA;
b) providing a coding or non-coding ss cDNA strand from said cloned ds cDNA;
and then carrying out step' c), d), e) and f) in the order decf or cedf:
c) hybridizing to the non-coding strand at the juncture of the coding sequences for the leader region and variable region a first oligonucleotide primer having an initiation codon for defining the initiation site to produce a first duplex, enzymatically treating this duplex to elongate said first oligonucleotide primer in its 5'-3' direction complementary to said non-coding ss cDNA, and digesting said non-coding ss cDNA in the other direction up to the sequence complementary to said first oligonucleotide primer, to produce N-terminus defined ds cDNA;
d) hybridizing to the coding strand at the DNA sequences coding for the juncture of the variable region and the constant region a second oligonucleotide primer that includes a stop anti-codon to produce a second duplex, enzymatically treating this duplex to elongate the second oligonucleotide primer in its 5'-3' direction complementary to said coding strand and digesting said coding ss cDNA in the other direction up to the sequence complementary to said second oligonucleotide primer, to produce C-terminus tailored ds cDNA; e) cloning the resulting ds cDNA with its C- or N-terminus defined; separating the resulting ds cDNA with its C- or N-terminus defined into coding and non-coding strands; and using said coding strand if step d) follows but said non-coding strand if step c) follows;

and f) cloning the resulting N- and C-terminus tailored ds cDNA; and inserting said N- and C-terminus tailored ds cDNA into an expression vector or plasmid with said coding sequence in proper relationship with transcriptional and translational regulatory signals.

A preferred embodiment of this method comprises:

A) preparing ds cDNA coding for a light or heavy chain of an immunoglobulin, each chain being composed of a constant region and a variable region, said variable regions having about 95 to 125 amino acids;
by the steps of isolating mRNA that codes for said chain, reverse-transcribing said mRNA to produce ss cDNA, synthesizing a strand complementary to said ss cDNA by means of DNA polymerase to produce ds cDNA having a coding strand coding for said light or heavy chain, wherein said coding strands include DNA sequences that code for leader sequence, variable region and constant region of said immunoglobulin in the 5'-3' direction of said coding strand, and cloning said ds cDNA;
B) removing at least a portion of the DNA coding for the regions flanking said variable region of said light or heavy chain by separating the cloned ds cDNA into coding and non-coding strands;
hybridizing to the non-coding strand a first oligonucleotide primer having an initiation codon for defining the initiation site for expression of a variable region, said first oligonucleotide primer being complementary to the sequence coding for the N-terminus of the leader region or partially complementary to the DNA sequence coding for the juncture of the leader region and variable region, having a non-complementary initiation codon about at said juncture, to produce a first duplex, enzymatically treating the resulting duplex to elongate the first oligonucleotide primer in its 5'-3' direction complementary to said non-coding ss cDNA, and digesting said non-coding ss cDNA in the other direction up to the sequence complementary to said first oligonucleotide primer, to produce N-terminus defined ds cDNA;
cloning the resulting N-terminus defined ds cDNA;

separating the resulting N-terminus defined ds cDNA into coding and non-coding strands;
hybridizing to the coding strand a second oligonulceotide primer that includes a stop anti-codon but is otherwise complementary to the sequence at about the juncture of said variable region and said constant region to produce a second duplex, said stop anti-codon being at said juncture and thereby introducing a stop codon at the terminus of said variable region, enzymatically treating the resulting duplex to elongate said second oligonucleotide primer in its 5'-3' direction complementary to said coding strand and digesting said coding ss cDNA in the other direction up to the sequence complementary to said second oligonucleotide primer, to produce N- and C-terminus tailored ds cDNA coding for the variable region of the light or heavy chain free of the constant region of said immunoglobulin;
cloning the resulting N- and C-terminus tailored ds cDNA;
and inserting said N- and C-terminus tailored ds cDNA into an expression vector or plasmid with said coding sequence in proper relationship with transcriptional and translational regulatory signals.

The first oligonucleotide primer may homoduplex with said non-coding strand at the N-terminus of said leader sequence; or may hybridize at about the juncture between said leader sequence and said variable sequence to introduce an initiation codon at the N-terminus of the DNA sequence coding for said variable region. At least one oligonucleotide primer may be only partially complementary to said cDNA strand.

The method may include the additional step, prior to said inserting, of ligating unique restriction linkers to said N- and C-terminus tailored ds cDNA and enzymatically cleaving said linkers to provide cohesive termini. The cloning after each hybridizing step may include the additional step of selecting clones having said first or second oligonucleotide sequence, isolating the DNA containing said ds cDNA and recloning said ds cDNA.

The principles and details of the present invention can be applied to the preparation of a transformed expression vector or plasmid which carries a ds DNA sequence that codes for only a desired part of a polypeptide chain of a protein or enzyme and is equipped for initiation and termination codons at the 5'- and 3'-termini respectively of said DNA sequence;
by a method comprising:
preparing ds cDNA from an m-RNA coding for said protein or enzyme;
removing nucleotide sequences from said ds cDNA superfluous to said desired part of said polypeptide chain and providing for initiation and termination codons at the 5'-and 3'-termini respectively of the DNA sequence to provide tailored ds cDNA encoding said desired part of said polypeptide chain,
and inserting said tailored ds cDNA into an expression vector for expression of said ds cDNA.

The foregoing features of the present invention, especially the steps a) to f), can be adapted accordingly.

An important feature of the present invention comprises a method for preparing a binding polypeptide which consists essentially of the amino acid sequence of at least a portion of the variable region of a light or heavy chain of an immunoglobulin specific for a predetermined ligand, said amino acid sequence having substantially the binding specificity of the analogous chain,
said method comprising:
preparing ds cDNA encoding at least one of said light or heavy chains from an mRNA coding for said chain;
removing nucleotide sequences from said ds cDNA superfluous to said variable region, and providing for initiation and termination codons at the 5'- and 3'-termini respectively of the DNA sequence to provide tailored ds cDNA encoding said variable region;
inserting said tailored ds cDNA into an expression vector for expression of said ds cDNA and transforming a host for said expression vector with said expression vector containing said tailored ds cDNA;
growing said transformed host, whereby said binding polypeptide of one of said light and heavy chains is expressed; and
isolating said binding polypeptide.

Another important feature of the present invention comprises a method for preparing a binding polypeptide which consists essentially of the amino acid sequence of at least a portion of the variable region of a light or heavy chain of an immunoglobulin specific for a predetermined ligand, said amino acid sequence having substantially the binding specificity of the analogous chain,
said method comprising:
growing a host transformed by a transformed expression vector or plasmid which carries a ds DNA sequence that codes for a variable region of a light or heavy chain of said immunoglobulin but lacks nucleotides coding for amino acid residues superfluous to said variable region and is equipped for initiation and termination codons at the 5'-and 3'-termini respectively of said DNA sequence.

The preparation of the rFv by hybrid DNA technology will nob be described in greater detail.

1. Isolation of appropriate DNA Sequences.

In preparing the DNA sequences, a source of the genes encoding the variable region will be required. The variable regions may be derived from IgA, IgD, IgE, IgG or IgM, most commonly from IgM and IgG. This can be achieved by immunizing an appropriate vertebrate, normally a domestic animal, and most conveniently a mouse. The immunization may be carried out conventionally with one or more repeated injections of the immunogen into the host mammal, normally at two to three week intervals. Usually three days after the last challenge, the spleen is removed and dissociated into single cells to be used for cell fusion to provide hybridomas.

The immunogen will be the antigen of interest, or where a hapten is present, an antigenic conjugate of the hapten to an antigen.

In order to prepare the hybridomas, the spleen cells are fused under conventional conditions employing a fusing agent, e.g. PEG6000, to a variety of inter- or intraspecies myeloma cells, particularly mouse cells such as SP-2/0, NS-1, etc. and then suspended in HAT selective media. The surviving cells are then grown in microtiter wells and immunologically assayed for production of antibodies to the determinant site(s) of interest.

Assays for antibodies are well known in the art and may employ a variety of labeled antigens or haptens, where the labels are conveniently radioisotopes, fluorescers, enzymes, or the like. Other techniques may also be employed, such as sandwich techniques involving two antibodies, one bound to a support and the other labeled. The cells from microtiter wells scored as positive are cloned either by limiting dilution or in soft agar. The resulting cloned cell lines are then propagated in an appropriate nutrient medium and, if necessary, may be stored frozen in liquid nitrogen.

After selection of a particular cell line providing a monoclonal antibody of interest, the cells are expanded. Conveniently, the cells may be grown to a density of about $1 \times 10^6$ cells/ml in a 1 liter culture. The cells are then harvested by centrifugation and lysed.

To obtain the desired DNA sequence, one can look to either the gene expressing the variable region or the messenger RNA, which expresses the variable region. The difficulty with employing genomic DNA is in juxtaposing the sequences coding for the variable region when these sequences are separated by introns. One must isolate the DNA fragment(s) containing the proper exons, excise the introns and then splice the exons in the proper order and orientation. Generally this will be difficult, so that the alternative technique employing the messenger RNA will be the method of choice.

Where the messenger RNA is to be employed, the cells will be lysed under RNase inhibiting conditions. Mature messenger RNA has the advantage that it is free of introns, so that the nucleotide sequence is continuous for the entire variable region. Difficulties with messenger RNA have been encountered, owing to incomplete reverse transcription, but these can be minimized. The first step is to isolate the messenger RNA. Conveniently, messenger RNA,which is polyadenylated, can be separated from other RNA on an oligo-(dT) cellulose column. The mixture of messenger RNA will be obtained free of other RNA. The presence of messenger RNAs coding for the heavy and light chain polypeptides of the immunoglobulins may then be assayed by hybridization with DNA single strands of the appropriate genes. Conveniently, the sequences coding for the constant portion of the light and heavy chains may be used as probes, which sequences may be obtained from available sources (see, for example, Early and Hood, Genetic Engineering, Setlow and Hollaender eds. Vol. 3, Plenum Publishing Corp., New York (1981), pages 157-188 ).

Whether the messenger RNA codes for the correct immunoglobulin may be determined by in vitro translation employing a rabbit reticulocyte cell-free extract (Pelham and Jackson, Eurp. J. Biochem. (1976) 66:247-256). The resulting translation product may then be isolated by employing antibodies specific for one or more of the regions of the chain of interest, for example, using rabbit anti(mouse IgG) where the chains are derived from mouse immunoglobulin.

The immunoprecipitate may be further analyzed by polyacrylamide gel electrophoresis, and the presence of complexes determined by using radiotagged receptors for antigen-antibody complexes, such as S. aureus protein A, Rf factor, or the like. In addition, RNA blot hybridization (resolution of the mRNA samples on agarose gel, transfer of the mRNA to nitrocellulose filter, baking at 80°C. and testing with radioactive probes) can be employed to further ensure that the correct messenger RNA is present.

The crude mixture of mRNA sequences containing the desired mRNA sequences will be treated as follows. In order to enhance the probability that full length cDNA is obtained, the method of Okayama and Berg, Mol. Cell. Biol. (1982) may be employed. Alternatively, the methods described by Efstradiadis and Villa-Komaroff (1979) in Genetic Engineering: Principles and Methods 1, Setlow and Hollaender, eds., New York, Plenum Press, pages 15-36, or Steinmetz et al. (1981) Cell 24:125-134,may be employed. The first strand of cDNA is prepared employing a virus reverse transcriptase in the presence of oligo-(dT)-primer. A

second strand may then be prepared employing reverse transcriptase, the Klenow fragment of DNA polymerase I or T4 polymerase. If necessary, the resulting ds cDNA may then be treated with a single-strand-specific nuclease such as SI nuclease for removal of single stranded portions to result in ds cDNA, which may then be cloned.

2. Preparation of Genes Coding For L-rFv and H-rFv and Introduction into an Expression Vector For Amplification.

A wide variety of vectors may be employed for amplification or expression of the ds cDNA to produce the light and heavy chains of the immunoglobulin. A vector having an appropriate restriction site is digested with the appropriate endonuclease. The ds cDNA obtained from the reverse transcription of the mRNA may be modified by ligating linkers, treatment with terminal transferase or other techniques to provide staggered (complementary) or blunt ended termini. The vectors may have one, two or more markers for selection of transformants. Desirably, the vector will have a unique restriction site in one of multiple markers, so that the transformants may be selected by the expression of one marker and the absence of expression of the other marker. Various markers may be employed, such as biocide resistance, complementation of an auxotroph, viral immunity, or the like.

An appropriate host, usually bacterial, e.g. E. coli, B. subtilis, S. cerevisiae, etc., is transformed according to conventional methods with the ds cDNA prepared from the mRNA, and the transformants are spread on agar plates and selected in accordance with the particular markers. The resulting colonies are screened, by restriction electrophoretic pattern, by hybridization to a labeled probe or by any other conventional means. See, for example, Hanahan and Meselson (1980) , Gene 10:63-67. One procedure employs colony hybridization, where the transformants are grown on a solid medium to produce colonies. Cells from the colonies are transferred to a nitrocellulose replica filter, the transferred cells incubated for further growth, lysed, dried and baked e.g. at 80 °C. The replica filter is then hybridized with appropriate radioisotope labeled probes. Probes for the determinant bonding sites present in the constant regions of a variety of mammalian immunoglobulins are readily available. The colonies may be probed according to the nature of the particular immunoglobulin or of the different determinant sites that may be present in the particular immunoglobulin.

The host colonies that hybridise with the probes, i.e. that have DNA coding for either the light or the heavy chain, are picked and then grown in culture under selective pressure. In order to maintain selective pressure, it is desirable that the vector which is employed have biocidal, particularly antibiotic, resistance. After sufficient time for expansion of the host, the host cells are harvested, conveniently by centrifugation. The hybrid plasmid DNA may then be isolated by known procedures (e.g. those of Gunsalus et al., J. Bacteriol. (1979) 140:106-133).

The isolated plasmid DNA is then characterized by restriction enzyme digestion and DNA sequence analysis. These analyses ensure that the isolated cDNA clones completely encode the variable region and, optionally, the leader sequences for the light or heavy chain of the desired immunoglobulin. Furthermore, by having a restriction map of the variable regions, leader sequences and flanking sequences, one can determine the appropriate restriction sites for excising a DNA fragment which will allow for appropriate modification of the DNA sequence for insertion into a vector and expression of the polypeptide of interest. Where no unique restriction site is available at an appropriate position in the flanking regions, partial digestion may be employed, with selection of fragments having the variable region and, optionally, the leader sequence intact. Where the 5' and 3' flanking regions are too extended, these can be shortened using Bal 31 to varying degrees by varying the period of digestion.

Furthermore, by knowing the DNA sequence of the coding strand in the region of the C-terminus of the heavy and light chain variable regions, a stop codon may be introduced at the C-terminus by the following procedure of in vitro mutagenesis. The cDNA is restricted with the appropriate enzyme(s) to provide a segment coding for the variable region with additional 5' and 3' flanking sequences. This segment is purified, for example by gel electrophoresis, gradient density centrifugation, etc. The desired segment is isolated and its two strands are dissociated, conveniently by boiling. Alternatively, the undesired strand of the intact cDNA-plasmid clone may be nicked and digested.

A synthetic, single-stranded oligonucleotide(DNA oligomer) is prepared, conveniently by synthesis, which will have a least about 12 nucleotides, more usually about 15 nucleotides, and will generally have fewer than about 50 nucleotides, usually fewer than 30 nucleotides, since a more extended oligomer is not required.

Where this synthetic DNA oligomer is used in heteroduplexing, the non-complementary nucleotides will usually be flanked by at least about three, more usually at least about six nucleotides complementary to the

hybridized strand. The heteroduplexing DNA oligomer will be complementary to the sequence at or about a significant juncture i.e. between the leader sequence and the variable region or the variable region and the constant region. The DNA oligomer will be substantially complementary to the coding ("sense") strand of the variable-region sequence but will be altered to encode a termination codon at the C-terminus of the variable region. That is, the DNA oligomer will be complementary to the coding strand except at or about the amino acid which is involved at the juncture of the variable region and the D-, J-or C regions of the light and heavy chains, particularly at or intermediate the D- or J- regions or intermediate the J-region, or at the J-region and C-region juncture. It is intended that there will be some variation in the polypeptides which are prepared, so far as extending beyond the variable domains or not including all of the amino acids at the C-terminus of the variable region.

A excess amount of the DNA oligomer is combined with the denatured strands of the restriction fragment under sufficiently stringent hybridization conditions. Thus, the DNA oligomer specifically heteroduplexes to the complementary portions of the coding strand, while providing one or more stop codons to ensure the termination of expression at the desired amino acid at the C-terminus.

After sufficient time for hybridization at the desired level of stringency, sufficient amounts of the four deoxynucleotides are added in conjunction with the Klenow fragment of DNA polymerase I. A strand complementary to the coding sequence of the variable-region and any 5'-flanking sequence is synthesized by enzymatic elongation of the primer resulting in a sequence complementary to the strand to which the DNA oligomer is bound. The single-stranded DNA sequence on the coding strand located 3' to the region hybridized to the synthetic oligonucleotide is degraded by the 3'-5' exonuclease activity of the DNA polymerase. In this manner, ds cDNA is obtained which specifically codes for the variable-region and upstream flanking regions associated with the light and heavy chains. Each of the heavy and light chains is encoded to terminate expression at a predetermined codon in the V, D or J region.

The resulting heteroduplexed blunt-ended ds cDNA fragments are then employed for preparation of otherwise homoduplexed ds cDNA coding for the light and heavy variable regions with the stop codons at the desired sites. Conveniently, the blunt ended fragments are either modified as described previously, e.g. joined to linkers which code for restriction sites which are absent in the variable region sequences, or tailed, e.g. with polyG or polyC tails,or used directly for insertion. Thus a fragment, after being joined to a restriction site linker, can be inserted into an appropriate vector having complementary termini, and then when desired can be recovered by restriction at the linker sites. The linkers are joined to the blunt-ended fragments with an appropriate ligase e.g. T4 ligase, and the resulting ligated fragment is restricted to provide a shorter fragment with cohensive ends, which is annealed to the complementary ends of a vector.

This vector provides for amplification and convenient isolation of transformants having the variable region coding sequence insert. Numerous vectors for amplification in bacteria or other hosts exist such as pBR322, pSC101, pRK290, 2$\mu$-plasmid, etc. Annealing the shorter fragment with the cohesive ends to the vector will provide a hybrid plasmid that has complementary DNA sequences except where the DNA oligomer introduced heteroduplexing; here the DNA sequences will be mismatched. This hybrid plasmid (containing mismatched sequences) will replicate in the host to generate two different plasmid molecules, one with the original sequence and one with the "tailored" or "site mutated" sequence derived from the synthetic DNA oligomer. Therefore, each transformant colony is grown in small (approximately 2ml) culture, and the plasmid DNA is isolated in accordance with known procedures and used for a second cycle of transformation to provide individual clones replicating the "tailored" sequence. The clones may be screened by filter blot hybridization, or by probing with a labeled synthetic DNA oligonucleotide, e.g. the synthetic DNA oligomer employed in "tailoring" the variable region sequence, or by some other convenient technique. In this way plasmids are obtained having ds cDNA flanked by appropriate restriction sites and having a stop codon at a predetermined site.

The 3'-terminus of the coding strand (defining the C-terminus amino acid) has been defined, and the 5'-region of the coding strand (defining the N-terminus of the polypeptide) is next defined. Of course, the particular order in which the two termini are modified is primarily one of convenience; indeed, the two termini can even be modified simultaneously, where primer repair is used at the 5'-end of the coding strand in conjunction with site mutation at the 3'-end.

Different strategies may be developed, depending upon the nature of the host in which expression is to be obtained, and whether the host removes the leader sequence after recognising it as a secretory signal for secretion of the polypeptide. If the host cannot do this, then a DNA sequence coding for the leader sequence must be removed to provide a start codon at the 5'-terminus of the sequence of the coding strand coding for the variable region; then the shortened sequence can be inserted into an expression vector in such a way as to be under the control of a predetermined promoter and ribosomal start site.

Based on the sequence of the leader region or the sequence coding for the N-terminus of the variable

region, different oligonucleotides for homo- or heteroduplexing can be prepared.

Where the leader sequence is retained, primer repair is employed to remove the 5'-flanking sequence of the coding strand. When the primer repair of the N-terminus is performed simultaneously with the C-terminus mutagenesis, the coding strand, duplexed with the oligonucleotide, is treated with DNA polymerase, and the resulting partial double-stranded DNA is then treated with a 5'-3'-single strand exonuclease to remove the 5'-flanking region and with a ligase to provide for covalent linking of the replicated strand to the N-terminus oligonucleotide.

Where the leader sequence is to be removed, in vitro mutagenesis is employed to introduce an initiating codon (ATG, for N-formyl-methionine [f-met]) at the N-terminus of the DNA sequence coding for the variable region.

Alternative strategies may be employed for recovering the desired ds cDNA and performing the in vitro mutagenesis. If useful restriction sites are distant from the coding regions, the plasmid may be digested with the appropriate restriction endonuclease, followed by digestion with a double-strand exonuclease e.g. Bal 31. The resulting ds cDNA may be cloned and the proper sequence selected and modified, as appropriate, as described above. If the non-coding flanking region at the 5'-terminus of the coding strand is too long, it may be digested with an endonuclease where a convenient restriction bite is available, or by digestion with an exonuclease e.g. Bal 31.

By repeating the above described procedure for modifying the 3'-terminus, except that the oligonucleotide is now complementary to the non-coding (nonsense) strand and includes an initiation codon at the 5'-end (primer repair) or within the oligonucleotide (in vitro mutagenesis), the 5'-terminus of DNA sequence encoding the variable regions may be tailored. Normally, the oligonucleotide homoduplexes for primer repair and heteroduplexes for in vitro mutagenesis. In this way, "tailored" ds cDNA is obtained which has start and stop codons properly positioned to define the variable regions of both the light and heavy chains of immunoglobulins. The resulting blunt ended ds cDNA may be modified, e.g. by addition of linkers, to provide complementary termini for insertion into an expression vector in proper spacing to the regulatory signals which are ligated to the ds cDNA or are present in the vector.

The ds cDNA is now ready for insertion into a vector for expression. As distinguished from the earlier vectors, which were solely concerned with replication of the ds cDNA, the vector which is employed at this stage requires the presence of the regulatory signals for transcription and translation.

A vector is chosen having an appropriate promoter, as well as other transcriptional regulatory signal sequences, such as an operator, attenuator, or activator. Also, the vector will have been at least partially sequenced, so as to determine the presence of at least one insertion site for introduction of the ds cDNA coding for the variable regions at a site under the control of the regulatory signals.

Besides transcriptional regulatory signals there are, as already indicated, translational regulatory signals, primarily the ribosomal binding site (Shine-Dalgarno sequence, "S-D") and the initiation codon ("f-met codon"). The S-D sequence and the initiation codon must be in the proper spacing, generally spaced apart by from about 3 to 12 base pairs. The S-D sequence may be present on the vector in appropriate juxtaposition to an insertion site or may be joined to the variable region coding sequence, for example, by ligation of an oligonucleotide providing the S-D sequence and an appropriate restriction site upstream from the S-D sequence. Alternatively, the S-D sequence may be introduced by in vitro mutagenesis, as previously described. The coding sequence must be in frame with the initiation codon.

In choosing the different strategies, considerations include the presence or absence of particular restriction sites in the variable region coding sequence and flanking regions; the availability of vectors which allow for insertion of the ds cDNA sequence into the vector and expression of the variable region polypeptide; the availability of useful shuttle vectors; the availability of hosts which permit expression and isolation in good yield; and the ability of the host to recognize such signals as secretory signals to cleave off the leader sequence. Therefore, in each situation with each different idiotype, it will be necessary to make a restriction map of at least portions of the DNA sequence coding for the variable region and the flanking regions.

Where the termini of the vector and sequence to be inserted are the same, it will be necessary to check that the sequence has been inserted in the correct rather than the incorrect orientation. By mapping the resulting cloned plasmids after insertion, one can select those plasmids having the variable region sequence in the proper orientation.

The above strategy allows for a number of important advantages. The polypeptide chains are prepared as a homogeneous composition containing identical sequences and chain lengths. The polypeptides forming the rFv will be free of sugars and, by virtue of their homogeneous and unglycosylated character, may be more uniformly labeled or modified. In this way products are obtained of uniform and reproducible properties. Thus, the products may be reliably administered to a mammalian host with relatively slight

concern for unexpected responses that could be caused by a heterogeneous spectrum of products.

The following Example illustrates the present invention but in no way limits it:

EXPERIMENTAL

The following Example description will be directed to the dinitrophenyl ligand as an example of a typical ligand. It is to be understood that the subject process will be useful for any ligand, although, owing to the wide variety of idiotypes involved, at various stages the process may have to be modified slightly to accommodate the presence of a particular restriction site or other unique feature.

Example

Preparation of Monoclonal Antibodies for Dinitrophenyl (DNP)

Into an aqueous buffered medium at about pH 10.5 is introduced 10mmoles 2,4-dinitrobenzene sulfonate (c.f. Eisen et al., J.A.C.S., 75 (1953), 4583) and 0.01mmole of keyhole limpet hemocyanin and the mixture rocked for 20 hours at room temperature. The solution is then dialyzed against successive changes of 0.6M NaCl and the residue is isolated to be used for immunization.

This DNP immunogen, 100 μg, is Combined as an emulsion with 0.1ml complete or incomplete Freund's adjuvant and 0.1ml PBS per dose. Each of 6 BALB/c mice is injected with four such doses at weekly intervals, each dose distributed intraperitoneally as well as subcutaneously into foot pads and into inguinal areas. The first injection is given with complete and the remaining three with incomplete Freund's adjuvant. Three days after the last injection, the mice are sacrificed and the spleens are isolated and used for formation of monoclonal antibodies.

The fusion is performed by combining $3x10^7$ Sp2/0-Ag14 myeloma cells (Shulman et al. (1978) Nature 276:269-270) and $5x10^7$ spleen cells and the mixture centrifuged at 200g for 5min and resuspended slowly in 0.6ml 50% PEG 1500 in Dulbecco's modified Eagle's medium (Flow). After 1 min. at 37° C, 20ml of R medium (RPMI 1640 medium (Gibco) supplemented with 30mM Hepes (Flow)) is added slowly. The cells are then centrifuged and resuspended in 20ml of R medium supplemented with 10% fetal calf's serum (Gibco) (RF medium) and 0.2ml of this suspension is then distributed to each of 200 wells containing 0.8ml RF medium. One hundred of these wells also contain $2x10^5$ mouse peritoneal exudate cells. After 24 hours' incubation, 1ml RF supplemented with HAT medium is added to each well. Every 2-3 days, 1ml or the medium is replaced with fresh RF+HAT. After two weeks, the cells demonstrating growth are tested for immunoglobulin production employing $^{35}$S-2,4-dinitrophenylsulfenamide of lysine. Clones showing specific activity are cloned by plating in soft agar to provide anti-DNP as required.

Alternatively, one may use the method described by Herzenberg et al. (1980) J. Exp. Med. 151: 1071-1087. In this method, DNP substituted bovine serum albumin is added to individual wells in a microtiter plate in an RIA diluent (1% BSA, 0.005M EDTA and 0.1% $NaN_3$ in PBS pH7.6) (50μl, 0.05mg/ml) and the mixture is incubated for 1 hour in the wells. Test or standard antisera at various dilutions are then added to coated wells (20μl/well) and incubated For 1h. After washing three times with the RIA diluent, $^{125}$I-labeled anti-mouse immunoglobulin (approximately $2x10^5$ cpm/well) is added and the mixture in incubated for 1h. Plates are then washed 3x with the RIA diluent, dried and evaluated by autoradiography.

Both these methods of detecting the presence of the desired antibody are well known. The cells are then cloned either by limiting dilution or in soft agar and the resulting cloned cell lines are propagated and stored frozen in liquid nitrogen for use as required.

Cells from one of the positive cloned cell lines are grown to a density of about $1x10^6$ cells/ml in a one liter culture. The cells are harvested by centrifugation and 1 gram of the cells is dropped into 16ml of guanidinium thiocyanate stock solution (4M, 50g of guanidinium thiocyanate with 0.5g of sodium N-lauryl sarcosine, 2.5ml of 1M sodium citrate, pH7.0, 0.7ml of 2-mercaptoethanol and 0.5ml of Sigma 30% Antifoam A, and the volume brought to 100ml at room temperature) in & 55ml Potter-Elbehjem homogenizer tube and is immediately homogenized for 30-60 secs. at full speed with an 18mm diameter Tissumizer homogenizer (Tekmar Industries). The resulting homogenate is centrifuged for 10min. at 8,000rpm in a Sorval HB4 swinging bucket rotor at 10° C. The supernatants are decanted into & flask, mixed with 0.024 volume (relative to the original volume of homogenizing buffer) of 1M acetic acid to lower the pH from 7 to 5 and 0.75 volume of absolute ethanol. The flask is capped and thoroughly shaken and then stored at -20° C overnight, and the material is sedimented by centrifugation for 10min at -10° C at 6,000rpm in an HB4 rotor.

The resulting firm pellet is isolated and resuspended by vigorous shaking in 0.5 volume buffered

guanidine hydrochloride stock solution (7.5M, neutralized and then buffered with 0.25 volume of 1M sodium citrate, pH7.0, 5mM in dithiothreitol). The samples are briefly warmed in a 68° C water bath to ensure complete dispersion of the pellets, followed by precipitation by adding 0.025 volume (relative to the amount of guanidine hydrochloride) of 1M acetic acid in 0.5 volume ethanol. The solution is maintained for at least 3h at -20° C and then centrifuged; the resulting pellet is resuspended in guanidine hydrochloride and reprecipitated as described above. The reprecipitated material is centrifuged for 5min at 6,000rpm and thereafter all reactions are carried out under sterile conditions.

The final pellets are dispersed in ethanol at room temperature, triturated to extract excess guanidine hydrochloride and then centrifuged for 5min at 6,000rpm. The ethanol is evaporated with a stream or nitrogen and the RNA pellets dissolved with vigorous shaking in 1ml of sterile water per g. of original cells. After centrifugation for 10min at 13,000rpm at 10° C, the supernatant containing the RNA is decanted. To ensure the complete extraction of all the RNA, the insoluble material is reextracted twice with 0.5ml of sterile water, the extract centrifuged for 10min at 13,000rpm at 10° C and the aqueous solutions combined, mixed with 0.1 volume of 2M potassium acetate/acetic acid, pH5, and 2 volumes of ethanol and left overnight at -20° C.

The RNA is sedimented from the ethanol suspension by centrifugation for 20min at 20,000rpm at -10° C in Corex tubes in an HB4 rotor. The resulting pellets are thoroughly washed with 95% ethanol, dried with nitrogen and dissolved in 1ml/g cells of 10mM Tris buffer pH 7.5, 1mM EDTA, 0.2% SDS. After dissolution of the RNA pellet, 1/9 volume of 5M NaCl is added, and the solution applied to an oligo(dT)column (about 0.5g dry weight, T3 grade, Collaborative Research). The column is washed extensively with 0.5M NaCl, 10mM Tris, 1mM EDTA, pH 7.5,0.2% SDS, and then eluted with 10mM Tris, 1mM EDTA, pH 7.5, 0.05% SDS. The elution profile is monitored at $\lambda_{260}$. The UV absorbing fractions are pooled and precipitated by addition of acetic acid/sodium acetate buffer, pH 5, and 2.5 volumes of ethanol. The dried pellet is dissolved in 50$\mu$l (1 vol.) 10mM Tris, pH 7.5, 1mM EDTA, and 9 vol. DMSO added, immediately followed by 1 vol. of buffered 1M LiCL (1M LiCl, 50mM EDTA, 2.0% SDS, 10mM Tris, pH 6.5). This solution is heated at 55° for 5min, 100 vol. of binding buffer added, and then reapplied to the oligo(dT) cellulose column, equilibrated with binding buffer (0.5M NaCl, 10mM Tris, 1mM EDTA, 0.2% SDS) and eluted as before.

The presence of messenger RNA encoding the monoclonal immunoglobulin heavy and light chain polypeptides is verified by hybrid selection employing DNA clones of the appropriate heavy and light chain genes from sources described in Early and Hood, Genetic Engineering (1981) Vol. 3, Setlow and Hollander, Plenum Publishing Corp., pages 157-188. DNA probes can be prepared by synthesis, based on published amino acid sequences or published DNA sequences or obtained from a variety of sources reported in Early and Hood, supra. The DNA probes are denatured, neutralized and bound to nitrocellulose filter paper (Schleicher and Schuell BA-85-R 597) according to the method of Southern, J.Mol. Biol. (1975) 98:503-517, in 10x conc. standard citrate. (See also U.S.P. No. 4,302,204.) The probes are hybridized to 30 $\mu$g of the messenger RNA in 65% formamide/10mM Pipes buffer,pH6.4/0.4M NaCl in a final volume of 100 $\mu$l at 50° C for 2h. The reaction mixture is spun for 10sec. in a Microfuge, vortexed, spun again and then gently vortexed to resuspend the filters. The mixture is incubated at 50° C for about 1h with mild agitation. The reaction mixture is then removed and the filters are washed ten times in 1ml 0.15M NaCl/0.15M Na citrate/0.5% $NaDodSO_4$, while maintaining the wash buffer at 60° C. After each addition of wash buffer, the tubes are vortexed for several seconds. The filters are then washed twice with 1ml 10mM Tris, pH 7.8, 2mM EDTA, the tubes being incubated at 60° C for 5min and the solution then removed by aspiration.

RNA is eluted from the RNA-DNA hybrid by boiling the filters for 60sec in 300 $\mu$l of double distilled, sterile water and then quick-freezing in a methanol/dry ice bath. After thawing on ice, the water (containing the RNA) is transferred to a second tube and brought to a final concentration of 0.2M of sodium acetate, and 20 $\mu$g of calf thymus tRNA is added. The RNA is precipitated with 2.5 volume of ethanol at -20° C and immediately prior to translation the RNA is pelleted at 12,000g for 10min at 4° C, the pellet washed twice with 70% ethanol and then dried under reduced pressure.

The eluted mRNA is now translated in vitro with rabbit reticulocyte cell-free lysate, e.g. that of the commercially available translation kit from New England Nuclear, whereafter protein synthesis may be detected according to instructions with the kit.

Aliquots are then incubated with monoclonal antibodies in substantial excess to the amount of expression product in the in vitro translation lysate. The anibody:antigen complex is then precipitated with fixed S. aureus and the precipitates are washed three times in 0.05M Tris, pH8.3, 0.45M NaCl in 0.5% NP40, boiled in 0.01M sodium phosphate buffer, pH7.5, containing 1% B-mercaptoethanol and then electrophoresed on 5-20% gradient SDS-polyacrylamide gels at 125V until the bromophenol blue marker runs off the end of the gel and for one further hour. The gels are then dried, fixed and autoradiographed on Kodak X-R film to establish the presence of messenger RNA coding for immunoglobulin light and heavy

chains. This messenger RNA mixture is then employed to prepare a library of double stranded cDNA by the method of Okayama and Berg, Molecular and Cellular Biology, Feb. 1982, 161-170. First, the preparation of vector primer and oligo dG-tailed linker DNA will be described.

400 $\mu$g of pBR322-SV40 (map units 0.71-0.86) DNA are digested at 37° with 700 units of KpnI endonuclease in a reaction mixture (0.4ml) containing 5mM Tris-HCl (pH 7.5), 6mM $MgCl_2$, 6mM NaCl, 6mM 2-mercaptoethanol and 0.1mg/ml bovine serum albumin (BSA). After 5hrs. the digestion is terminated with 40 $\mu$l of 0.25M EDTA (pH 8.0) and 20 $\mu$l of 10% SDS; the mixture is extracted (to remove protein) with water-saturated phenol-$CHCl_3$ (1:1) (hereafter referred to as phenol-$CHCl_3$) and the DNA is precipitated with ethanol.

Homopolymer tails averaging 60, but not more than about 80, dT residues per end are added to the KpnI endonuclease-generated termini with calf thymus terminal deoxynucleotidyl transferase as follows: the reaction mixture (0.2ml) contains as buffer 140mM sodium cacodylate-30 mM Tris-HCl (pH 6.8), 1mM $CoCl_2$, 0.1mM dithiothreitol, 0.25mMdTTP, the KpnI endonuclease-digested DNA and 400 units of the terminal deoxynucleotidyl transferase. After 30 minutes at 37°C the reaction is stopped with 20 $\mu$l of 0.25M EDTA (pH 8.0) and 10 $\mu$l of 10% SDS and the DNA is recovered after several extractions with phenol-$CHCl_3$ by ethanol precipitation. The DNA is then digested with 17 units of HpaI endonuclease in 0.2ml containing 10mM Tris-HCl (pH 7.4), 10mM $MgCl_2$, 20mM KCl, 1mM dithiothreitol and 0.1mg/ml BSA for 5hrs at 37°C.

The large DNA fragment, which contains the origin of pBR322 DNA replication and the gene conferring ampicillin resistance, is purified by agarose (1%) gel electrophoresis and is recovered from the gel by a modification of the glass powder method (Vogelstein and Gillespie, PNAS USA (1979) 76:615-619).

The dT-tailed DNA is further purified by adsorption and elution from a oligo dA-cellulose column as follows: The DNA is dissolved in 1ml of 10mM Tris-HCl (pH 7.3) buffer containing 1mM EDTA and 1M NaCl, cooled to 0° and applied to an oligo dA-cellulose column (0.6 x 2.5 cm) equilibrated with the same buffer at 0°. The column is washed with the same buffer at 0° and eluted with water at room temperature. The eluted DNA (14 $\mu$g) is precipitated with ethanol and dissolved in 100 $\mu$l of 10mM Tris-HCl (pH 7.3) with 1mM EDTA.

The oligo dG-tailed linker DNA is prepared by digesting 100 $\mu$g of pBR322-SV40(map units 0.19-0.32) with 120 units of PstI endonuclease in 0.2ml containing 6mM Tris-HCl (pH 7.4), 6mM $MgCl_2$, 6mM 2-mercaptoethanol, 50mM NaCl and 0.1mg/ml BSA. After 1.5hrs at 37° the reaction mixture is extracted with phenol-$CHCl_3$ and the DNA is precipitated with alcohol. Then, tails of 10-15 dG residues are added per end with 60 units of terminal deoxynucleotidyl transferase in the same reaction mixture (50 $\mu$l) described above, except that 0.1mM dGTP replaces dTTP. After 20 minutes at 37°C the mixture is extracted with phenol-$CHCl_3$, and the DNA is precipitated with ethanol and digested with 50 units of HindIII endonuclease in 50 $\mu$l containing 20mM Tris -HCl (pH 7.4), 7mM $MgCl_2$, 60mM NaCl and 0.1mg/ml BSA at 37° for 1 hr. The small oligo dG-tailed linker DNA is purified by agarose (1.8%) electrophoresis and recovered as described above.

The reaction mixture (10 $\mu$l) contains 50mM Tris-HCl (pH 8.3), 8mM $MgCl_2$, 30mM KCl, 0.3mM dithiothreitol, 2mM each dATP, dTTP, dGTP, and $^{32}$P-dCTP (850 cpm/pmol), 0.2 $\mu$g of the mRNA (about 2-3 fold excess over primer ends), 1.4 $\mu$g of the vector-primer DNA (0.7 pmole primer end) and 5 units of reverse transcriptase. (The molar ratio of polyA mRNA to vector-primer DNA ranges from about 1.5:1 to 3:1.)

cDNA synthesis is initiated by the addition of the reverse transcriptase and continued at 37° for 20min. By this time the rate of dCTP incorporation levels off and more than 60% of the primer is utilized for cDNA synthesis. The reaction is stopped with 1$\mu$l of 0.25M EDTA (pH 8.0) and 0.5 $\mu$l of 10% SDS; 10 $\mu$l of phenol-$CHCl_3$ is added and the solution vortexed vigorously and then centrifuged. 10 $\mu$l of 4M ammonium acetate and 40 $\mu$l of ethanol are added to the aqueous phase, and the solution is chilled with dry ice for 15min, warmed to room temperature with gentle shaking to dissolve unreacted deoxynucleoside triphosphates that precipitate during chilling, and centrifuged for 10min in an Eppendorf microfuge. The resulting pellet is dissolved in 10 $\mu$l of 10mM Tris-HCl (pH 7.3) and 1mM EDTA, mixed with 10 $\mu$l of 4M ammonium acetate and reprecipitated with 40 $\mu$l of ethanol, and then rinsed with ethanol.

The pellet containing the cDNA:mRNA plasmid is dissolved in 15 $\mu$l of 140mM sodium cacodylate-30mM Tris-HCl (pH 6.8) buffer containing 1mM $CoCl_2$, 0.1mM dithiothreitol, 0.2 $\mu$g of poly A, 66$\mu$M $^{32}$P-dCTP (6000 cpm/pmol) and 18 units of terminal deoxynucleotidyl transferase. The reaction is carried out at 37° for 5min to permit the addition of 10 to 15 residues of dCMP per end and then terminated with 1.5 $\mu$l of 0.25M EDTA (pH 8.0) and 0.75 $\mu$l of 10% SDS. The mixture is extracted with 15 $\mu$l of phenol-$CHCl_3$ and mixed with 15 $\mu$l of 4M ammonium acetate, the DNA is precipitated and reprecipitated with 60 $\mu$l of ethanol and the final pellet rinsed with ethanol.

This pellet is dissolved in 10 $\mu$l of buffer containing 20mM Tris-HCl (ph 7.4) 7mM $MgCl_2$, 60mM NaCl and 0.1mg/ml BSA and then digested with 2.5 units of HindIII endonuclease for 1hr at 37°C. The reaction is

terminated with 1 μl of 0.25M EDTA (pH 8.0) and 0.5 μl of 10% SDS, the mixture is extracted with phenol-$CHCl_3$, 10 μl of 4M ammonium acetate is added and the DNA is precipitated with 40 μl of ethanol. The resulting pellet is rinsed with ethanol and dissolved in 10 μl of 10mM Tris-HCl (pH 7.3) and 1mM EDTA, and 3 μl of ethanol are added to prevent freezing during storage at -20° C.

1 μl of the HindIII-endonuclease-digested oligo dC-tailed cDNA:mRNA plasmid (0.02 pmol) is incubated in a mixture (10 μl) containing 10mM Tris-HCl (pH 7.5), 1mM EDTA, 0.1M NaCl and 0.04 pmol of the oligo dG-tailed linker DNA (this amount is a one-fold molar excess over the quantity of the double strand cDNA:mRNA and of the fragment which remains as a result of the HindIII endonuclease digestion in the previous step) at 65° for 2min., followed by 42° for 30min. and then cooled at 0°. The mixture (10 μl) is adjusted to a volume of 100 μl containing 20mM Tris-HCl (pH 7.5), 4mM $MgCl_2$, 10mM $(NH_4)_2SO_4$, 0.1M KCl, 50 μg/ml BSA and 0.1mM β-NAD; 0.6 μg of E. coli DNA ligase is added and the solution is incubated overnight at 12°.

To replace the RNA strand of the double strand cDNA:mRNA, the ligation mixture is adjusted to contain 40μM of each of the four deoxynucleotide triphosphates, 0.15mM β-NAD, 0.4μg of additional E. coli DNA ligase, 0.3μg of E. coli DNA polymerase I, and 1 unit of E. coli RNase H. This mixture (104μl) is incubated successively at 12° C and room temperature for 1hr each to promote optimal repair synthesis and nick translation by PolI. The reaction is terminated by the addition of 0.9ml of cold 10mM Tris-HCl (pH 7.3), and 0.1ml aliquots are stored at 0° C.

Transformation is carried out using minor modifications of the procedure described by Cohen et al., PNAS USA (1972) 69:2110-2114. E. coli K12 (strain HB101) is grown at 37° C in 20ml standard L-broth to an optical density of 0.5 at $\lambda_{600}$. The cells are collected by centrifugation, suspended in 10ml of 10mM Tris-HCl (pH 7.3) containing 50mM $CaCl_2$ and centrifuged at 0° C for 5min. The cells are resuspended in 2ml of the above buffer and incubated again at 0° C for 5min.; then, 0.2 ml of the cell suspensions is mixed with 0.1ml of the DNA solution and incubated at 0° C for 15min. After the cells are kept at 37° C for 2min. and at room temperature for 10min., 0.5ml of standard L-broth is added, the culture incubated at 37° C for 30min., and then plated on nitrocellulose filters on agar plates containing 50 μg/ml ampicillin. After incubation at 37° C for 12-24 hrs., E. coli transformants are screened for the presence of the light and heavy chain cDNA according to the method of Grunstein and Hogness by in situ colony hybridization. Several thousand transformants are grown on three replica nitrocellulose filter discs, lysed with alkali and hybridized with the probes described previously for the constant regions of the heavy and light immunoglobulin chains. Clones of the genes coding for the heavy and light immunoglobulin chains are identified. Colonies that give positive hybridization signals are grown in one-liter of L-broth containing 50 μg/ml of ampicillin, and their plasmid DNAs are isolated by standard techniques (Gunsalus et al., J. Bact. (1979) 140:106-113).

The cells are lysed as described previously, the lysate cleared by centrifugation and the cleared lysate diluted with an equal volume of water. RNase A is added to 50 μg/ml and, after 1h at 37° C, the lysate is extracted with 0.3 volume of phenol saturated with TE buffer (10mM Tris-HCl, pH 7.9, plus 1mM EDTA). After centrifugation (16,000g, 4° C, 10min), the aqueous phase is removed and adjusted to 1M NaCl and the DNA precipitated with 2 volumes of ethanol. After several hours at -20° C, the DNA is pelleted by centrifugation (10,000g, 4° C 20min), dried and dissolved in TE buffer.

Each cDNA clone is then restriction mapped and sequence analysed by conventional techniques, so that a restriction map is obtained which allows for subsequent manipulation of the cDNA coding for the variable regions for cloning and expression. The methods of Maxam and Gilbert, Methods Enzymol. (1980) 65:499-560 and Sanger et al., J.Mol.Biol. (1980) 143:161-178 are used, respectively. Those cDNA clones for light chains and heavy chains encoding the complete variable region end leader sequences are selected for subsequent manipulation.

Illustrative of the subject method will be the isolation, sequencing and manipulation of the K-chain (light chain) of MOPC41 and the heavy chain of the myeloma S107.

The following is the sequence of the K-chain of MOPC41, where the sequences encoding the leader, variable region and constant region are separated by gaps, with only the first sixteen amino acids of the constant region indicated (Seidman et al., "Nature" (1979) 280: 370-375):

```
                        Met Asp Met Arg Ala Pro Ala
... TCA GGA CTC AGC ATG GAC ATG AGG GCT CCT GCA

Gln Ile Phe Gly Phe Leu Leu Leu Leu Phe Gln Gly
CAG ATT TTT GGC TTC TTG TTG CTC TTG TTT CAA GGT

Thr Arg Cys     Asp Ile Gln Met Thr Gln Ser Pro
ACC AGA TGT ... GAC ATC CAG ATG ACC CAG TCT CCA

Ser Ser Leu Ser Ala Ser Leu Gly Glu Arg Val Ser
TCC TCC TTA TCT GCC TCT CTG GGA GAA AGA GTC AGT

Leu Thr Cys Arg Ala Ser Gln Asp Ile Gly Ser Ser
CTC ACT TGT CGG CCA AGT CAG GAC ATT GGT AGT AGC

Leu Asn Trp Leu Gln Gln Glu Pro Asp Gly Thr Ile
TTA AAC TGG CTT CAG CAG GAA CCA GAT GGA ACT ATT

Lys Arg Leu Ile Tyr Ala Thr Ser Ser Leu Asp Ser
AAA CGC CTG ATC TAC GCC ACA TCC AGT TTA GAT TCT

Gly Val Pro Lys Arg Phe Ser Gly Ser Arg Ser Gly
GGT GTC CCC AAA AGG TTC AGT GGC AGT AGG TCT GGG

Ser Asp Tyr Ser Leu Thr Ile Ser Ser Leu Glu Ser
TCA GAT TAT TCT CTC ACC ATC AGC AGC CTT GAG TCT
```

```
Glu Asp Phe Val Asp Tyr Tyr Cys Leu Gln Tyr Ala
GAA GAT TTT GTA GAC TAT TAC TGT CTA CAA TAT GCT

Ser Ser Pro Trp Thr Phe Gly Gly Gly Thr Lys Leu
AGT TCT CCG TGG ACG TTC GGT GGA GGC ACC AAG CTG

Glu Ile Lys Arg     Ala Asp Ala Ala Pro Thr Val
GAA ATC AAA CGT ... GCT GAT GCT GCA CCA ACT GTA

Ser Ile Phe Pro Pro Ser Ser Glu Gln
TCC ATC TTC CCA CCA TCC AGT GAG CAG ...
```

The following is the nucleotide sequence of the heavy chain variable region of myeloma S107, with the leader, variable region and constant region separated by gaps, and only the first nine amino acids of the constant region depicted (Early et al. (1980), Cell. 19:981-992):

```
Met Lys Leu Trp Leu Asn Trp Val Phe Leu Leu Thr Leu
ATG AAG TTG TGG TTA AAC TGG GTT TTT CTT TTA ACA CTT

Leu His Gly Ile Gln Cys ... Glu Val Lys Leu Val Glu
TTA CAT GGT ATC CAG TGT     GAG GTG AAG CTG GTG GAA

Ser Gly Gly Gly Leu Val Gln Pro Gly Gly Ser Leu Arg
TCT GGA GGA GGC TTG GTA CAG CCT GGG GGT TCT CTG AGA

Leu Ser Cys Ala Thr Ser Gly Phe Thr Phe Ser Asp Phe
CTC TCC TGT GCA ACT TCT GGG TTC ACC TTC AGT GAT TTC

Tyr Met Glu Trp Val Arg Gln Pro Pro Gly Lys Arg Leu
TAC ATG GAG TGG GTC CGC CAG CCT CCA GGG AAG AGA CTG

Glu Trp Ile Ala Ala Ser Arg Asn Lys Ala Asn Asp Tyr
GAG TGG ATT GCT GCA AGT AGA AAC AAA GCT AAT GAT TAT

Thr Thr Glu Tyr Ser Ala Ser Val Lys Gly Arg Phe Ile
ACA ACA GAG TAC AGT GCA TCT GTG AAG GGT CGG TTC ATC

Val Ser Arg Asp Thr Ser Gln Ser Ile Leu Tyr Leu Gln
GTC TCC AGA GAC ACT TCC CAA AGC ATC CTC TAC CTT CAG

Met Asn Ala Leu Arg Ala Glu Asp Thr Ala Ile Tyr Tyr
ATG AAT GCC CTG AGA GCT GAG GAC ACT GCC ATT TAT TAC

Cys Ala Arg Asp Tyr Tyr Gly Ser Ser Tyr Trp Tyr Phe
TGT GCA AGA GAT TAC TAC GGT AGT AGC TAC TGG TAC TTC

Asp Val Trp Gly Ala Gly Thr Thr Val Thr Val Ser Ser
GAT GTC TGG GGC GCA GGG ACC ACG GTC ACC GTC TCC TCA

    Ala Lys Thr Thr Pro Pro Thr Val Tyr
... GCC AAA ACG ACA CCC CCA TCT GTC TAT ...
```

Based on the DNA sequencing and the restriction map, PstI sites are found at the -110 base pair of the coding strand and downstream from the termination site for the cDNA coding for the light chain, while convenient HindIII restriction sites are found upstream from the leader sequence and downstream from the termination site of the coding strand for the heavy chain. The leader sequences and coding sequences of the light and heavy chain variable regions are free of sequences recognized by the indicated endonucleases.

The isolated plasmid DNAs are digested with the respective endonucleases in accordance with the instructions of the supplied and the resulting fragments purified by electrophoresis on 2% agarose gels (Seakem), 15cm x 15cm x 0.2cm, at 100V for 2h. By employing markers, the band of the appropriate molecular weight is located and excised. The gel slice is placed directly into a 1.5ml Eppendorf tube, rapidly frozen and thawed twice in a Dry Ice-alcohol bath and then centrifuged 5min in the Eppendorf centrifuge (15,000 rpm) and the supernatant recovered. The supernatant is boiled in 6xSSC to denature the DNA and provide single strands, followed by cooling to 0° C.

Based on the DNA sequence, a DNA oligomer is prepared which is at least partially complementary to a short sequence of each of the non-coding ("anti-sense") strands of the variable region sequences of the light and heavy chains. The oligomer has an initiating codon (ATG, for N-formyl-methionine [f-met]) at its 5'-end and is complementary to the downstream nucleotides at the N-terminus of the leader sequence for primer repair; or has an f-met codon intermediate its ends and complementary sequences to the 3'-end of the coding sequence for the leader region and the 5'-end of the coding sequence for the variable regions for in vitro mutagenesis. The oligomers are readily prepared in accordance with the methods described by Itakura et al., J.Biol. Chem. (1975) 150:4592.

The following schemes depict the primer repair synthesis method for the light and heavy chains where the leader sequence is retained (a and b, respectively) and the in vitro mutagenesis method where the leader sequence is removed and an f-met codon introduced at the N-terminus of the coding sequence for the variable regions of the light and heavy chains (c and d, respectively). The extended lines represent long DNA chains, the short dashes the terminations introduced in these schemes.

```
a.        5'——————— ATG GAC ATG AGG GCT ——————————————3'

          3'———————TAC CTG TAC TCC CGA——————————————— 5'
                       |
                       |  1) Denature (i.e. separate the
                       |                 strands)
                       |  2) 5' - ATG GAC ATG AGG GC - 3'
                       v
                ATG GAC ATG AGG GC

          3'——————— TAC CTG TAC TCC CG———————————————5'
                       |
                       |  Klenow fragment + dNTPs (dATP,
                       |  dCTP, dGTP and dTTP)
                       v
          5' - ATG GAC ATG AGG GC ————————————3'
          3' - TAC CTG TAC TCC CG ————————————5'
```

```
b.  5'--------ATG AAG TTG TGG--------------------3'
    3'--------TAC TTC AAC ACC--------------------5'
                  |
                  |  1) Denature
                  |
                  |  2) 5' - ATG AAG TTG TGG
                  v
              ATG AAG TTG TGG

    3'--------TAC TTC AAC ACC--------------------5'
                  |
                  |     Klenow fragment + dNTPs
                  v
    5' - ATG AAG TTG TGG------------3'
    3' - TAC TTC AAC ACC------------5'


c. 5'----------GGT ACC AGA TGT GAC ATC CAG-------------3'
   3'----------CCA TGG TGT ACA CTG TAG GTC-------------5'
                   |  1) Denature
                   |  2) 5' - GGT ACC AGA TGG ACA TCC - 3'
                   v
            GG TAC CAG ATG GAC ATC C

   3'---------CC ATG GTC TAC CTG TAG G-----------------5'
                            \/
                            T
                  |
                  |Klenow fragment + dNTPs
                  v
    5' - GG TAC CAG ATG GAC ATC C--------3'
    3' - CC ATG GTC TAC CTG TAG G--------5'
                       \/
                       T
                  |1) PstI linker + T4 Polynucleotide
                  |     ligase
                  |
                  |2) PstI
                  |
                  |3) pBR322 PstI digest/T4 Poly-
                  |     nucleotide ligase
                  |4) Purify by cloning
                  v
    5' -  GG TAC CAG ATG GAC ATC C----------3'
    3' -  CC ATG GTC TAC CTG TAG G----------5'
```

d.

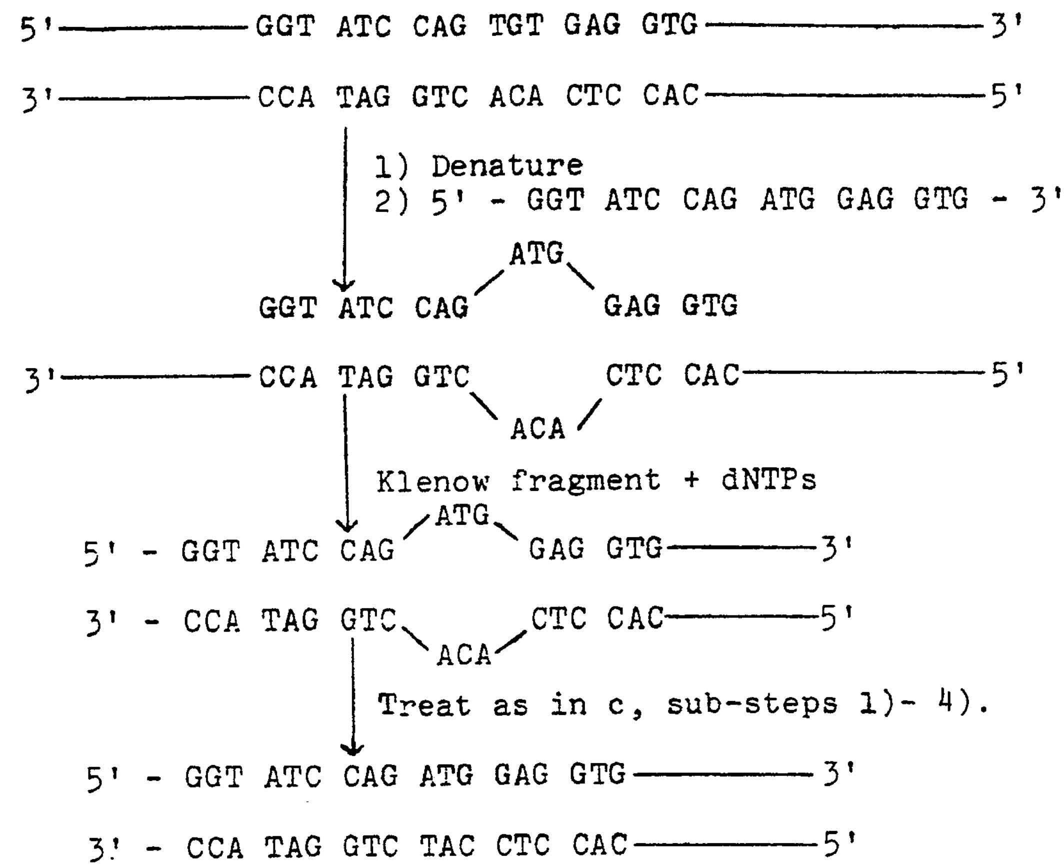


To 0.5 μg of the single stranded DNA is added 15pmole of 5'-phosphorylated oligonucleotide as described in a and b above in 38 μl of 200 mM of NaCl, 13mM Tris-HCl, pH7.5, 9mM Mg acetate, 20mM β-mercaptoethanol, the mixture boiled for 3min and immediately cooled to 0° C. To this is added 1μl of solution which contains the four deoxynucleoside triphosphates at 4mM, 0.1μl of 100mM adenosine triphosphate, and 1 μl (1 unit) of the Klenow fragment of DNA polymerase 1 (Boehringer Mannheim).

In this manner, strands coding for the 5'-leader sequence and coding sequence or just the coding sequence for the variable region are synthesized and the single-stranded DNA sequences in the 3'-direction of the template non-coding strand are degraded by the 3'-5'-exonuclease activity. As a result, for strands containing the leader sequence, homoduplexes are obtained for coding the leader sequence and variable regions for both the light and heavy chains, which are blunt ended, having an initiation codon at the 5'-end of the coding strand with the remaining DNA sequence in frame with the initiation codon.

To the resulting blunt ended duplex coding for the leader sequence and variable region of the chains, restriction enzyme linkers are ligated through the use of appropriate phosphorylated linkers, for example, PstI linkers, employing T4 polynucleotide ligase under conditions specified by the supplier. The vector pBR322 is cleaved with PstI to provide cohesive ends for linking to the modified cDNA.

Each cDNA is combined with the linear pBR322 having complementary termini. Equal molar amounts of the vector and cDNAs are combined in an annealing buffer essentially as described in Steinmetz et al. (1981) Cell. 24:125-134, and the annealed DNA used directly for transformation.

One ml of an overnight bacterial culture E. coli strain HB101 (Boyer and Roulland-Dussiox (1969) J. Mol. Biol. 41:459-472) is grown to $2x10^8$ cells/ml in L broth, pelleted by centifugation (Sorval SS34 rotor, 85,000rpm, 4° C, 5min) and washed in 0.5 volume cola 10mM $CaCl_2$. The cell pellet is resuspended in 0.5 volume cold 30mM $CaCl_2$. After 20 min or ice, the cells are again pelleted and resuspended in 0.1 volume cold 30mM $CaCl_2$. Then 0.20ml of the suspension is added to 0.1ml 30mM $CaCl_2$ containing the annealed plasmids and incubated on ice for 16min. Each transformation is then heated to 42° C for 75sec prior to the addition of 5ml L broth.

Transformed cultures are incubated at 37° C for 2hr. The transformants are then grown in agar plates containing M-9 minimal medium and 10 μg/ml tetracycline. Clones which grow on this medium are then transferred to agar plates having M-9 minimal medium and 40μg/ml of ampicillin. Those cells which are sensitive to ampicillin and resistant to tetracycline are then screened for the presence of plasmids having

the desired cDNA.

The selected clones are then grown in 2ml of nutrient culture for 18h. A 0.5ml aliquot is transferred to a 1.5ml Eppendorf tube for plasmid extraction. Manipulations are carried out at room temperature unless otherwise indicated. The tube is centrifuged for 15sec., the supernatant carefully removed with a fine-tip aspirator, and the cell pellet is thoroughly suspended in 100μl of a lysozyme solution containing 2mg/ml lysozyme,50mM glucose, 10mM EDTA, 25mM Tris-HCl (pH8.0).

After a 30min incubation at 0°C, 200μl of alkaline SDS solution (0.2N NaOH, 1% sodium dodecylsulfate) is added and the tube is gently vortexed. The tube is maintained for 5min at 0°C and then 150 μl of 3M sodium acetate (pH4.8) is added. After gently mixing by inversion for a few seconds, a clot of DNA forms and the tube is maintained at 0°C for 16min. After centrifugation for 5min. 0.4 ml of the supernatant is removed, transferred to a second centrifuge tube, 1ml cold ethanol added and the tube held at -20°C for 30min. The precipitate is collected by centrifugation for 2min and the supernatant removed by aspiration. The pellet is resuspended in 100 μl 0.1M sodium acetate, 200μl ethanol added, and after 10min at -20°C, the precipitate is again collected by centrifugation, and the pellet is dissolved in 50μl water.

Substantially the same procedure as described above is used for in vitro mutagenesis. With the primer repair synthesis, only one homoduplex is formed; with in vitro mutagenesis, a heteroduplex is initially formed which upon transformation and cloning results in two homoduplexes: the original gene sequence; and the modified or "tailored" gene sequence, which includes the change in sequence encoded in the oligomer.

As depicted in c and d, oligomers are prepared which introduce an initiation codon ATG, coding for f-met,at the N-terminus of the coding sequence for the variable regions.

The resulting plasmid DNA is isolated as described above and used again as described above for transformation. However, the resulting transformants are grown in small (2ml) culture for plasmid isolation. The plasmid DNA prepared from single transformant colonies arising from the second cycle of cloning is assayed by filter blot hybridization on nitrocellulose filters (Wallace et al. (1979) Nucleic Acids Research 6:3542-3556) probing with $^{32}$P-radio-labeled oligomers employed for the mutagenesis so as to ensure the isolation of the desired tailored homoduplexes of the cDNA. The clones having the tailored sequence are isolated and the plasmid DNA extracted for further prccessing at the 3'-end of the coding strand.

The cDNA coding for the variable regions can be excised by digestion with PstI. Repeating the technique described in the previous in vitro mutagenesis, where an ATG ("start") codon is introduced before the codon of the N-terminal amino acid of the mature polypeptide,"stop" codons are introduced at the C-terminus of the variable regions. Oligonucleotides are prepared as described previously having complementary sequences to the coding ("sense") strand of the variable-region cDNA.

The oligonucleotides and the schemes for inserting the stop codon at the end of the variable regions are depicted as follows. The introduction of the stop codon in the light chain is set forth in e, while the introduction of the stop codon in the heavy chain is set forth in f. In e and f, the stop codon (TGA) is underlined.

```
e.  5'————GAA ATC AAA CGT GCT GAT GCT TCA CC————3'
    3'————CTT TAG TTT GCA CGA CTA CGA CGT GG————5'
                  |
                  |  1) Denature
                  |  2) 3' - TTT GCA ACT ACG ACG TGG - 5'
                  ↓       GC
                        /    \
    5'————AAA CGT           T GAT GCT GCA CC————3'
       3' - TTT GCA————A CTA CGA CGT GG - 5'
                  |
                  |  Klenow fragment + dNTPs
                  ↓      GC
                       /    \
    5'————AAA CGT           T GAT GCT GCA CC - 3'
    3'————TTT GCA————A CTA CGA CGT GG - 5'
                  |
                  |  Treat as in c.
                  ↓
    5'————AAA CGT TGA TGC TGC ACC - 3'
    3'————TTT GCA ACT ACG ACG TGG - 5'
```

(TGA underlined in the final 5' strand.)

```
f.   5'————ACC GTC TCC TCA GCC AAA ACG ACA————3'
     3'————TGC CAG AGG AGT CGG TTT TGC TGT————5'
                 | 1) Denature
                 | 2) 3' - GAG GAG TAC TTT TGT CTG T - 5'
                 ↓                  GCC
     5'————ACC GTC TCC TCA       AAA ACG ACA————3'
            3' - G AGG AGT        TTT TGC TGT - 5'
                                 ACT
                 |
                 | Klenow fragment and dNTPs
                 ↓                  GCC
     5'————ACC CTC TCC TCA       AAA ACG ACA - 3'
     3' ———————— G AGG AGT        TTT TGC TGT - 5'
                                 ACT
                 | Treat as in c.
                 ↓
     5'——— ACC CTC TCC TCA TGA AAA ACG ACA - 3'

     3'———TGG GAG AGG AGT ACT TTT TGC TGT - 5'
```

The effect of the Klenow fragment and the four desoxynucleoside triphosphates is to degrade the 3'-end of the coding strand up to and including the first nucleotide unpaired with the oligomer and to extend the 3'-end of the oligomer complementary to the 5'-end of the coding strand; consequently, all of the sequence coding for the constant region, except for the few nucleotides paired with the oligonucleotide, is removed, but double strand DNA is built up in the opposite direction.

The heteroduplexes having the "tailored" sequences of the variable regions of the light and heavy chains are then ligated to PstI linkers, restricted with PstI endonuclease and inserted into the PstI site of pBR322. After cloning and recloning, the plasmids containing the tailored ds cDNA with the stop codons at the end of the variable regions are isolated and the sequences coding for the variable regions (which may also include the leader sequences) are excised from the pBR322 plasmid using the PstI restriction endonuclease and may now be used for expression of the polypeptide chains of the rFv.

In order to obtain expression of the variable regions, the plasmid pGMI (pVH253ΔtrpLE1413; Miozarri and Yanofsky, J. of Bacteriol. (1976) 133:1457-1466) is employed. The plasmid is modified to introduce a PstI site which provides for insertion of the sequences coding for the variable regions with the f-met codon ATG in proper position to the Shine-Dalgarno sequence. The following oligonucleotide sequence is prepared:

AGCTGCAGCTTTCGTT.

pCMI (10 μg) is nicked in one strand by digestion with EcoRI (Boehringer Mannheim, 1000 units) in 1ml of 100mM Tris-HCl, pH 7.2, 50mM NaCl, 5mM Mg acetate, 0.01 percent NP-40 and 150 μg/ml ethidium bromide at 37°C for 1h. The reaction mixture is brought to 10mM EDTA and then extracted with 3 x 10 volumes water-saturated isobutanol, 1 x phenol-$CHCl_3$, 2 x ether and 1 x isobutanol to reduce the volume to 0.1ml. After desalting by centrifugation through a 0.5ml Sephadex G-25 column, the DNA is recovered by precipitation with ethanol. Approximately 5 μg of the nicked DNA is incubated with 40 units of exonuclease III (BRL) in 20 μl of 10mM Tris-HCl, ph 7.5, 2mM $MgCl_2$ and 1mM β-mercaptoethanol for 90min at 37°c. The reaction is adjusted to 15mM Tris-HCl, pH 7.5, 7mM NaCl, 7mM $MgCl_2$, 7mM dithiothreitol. After adding 20 units of bacterial alkaline phosphatase (BRL) and 5 units of HinfI (BRL), digestion is continued for

30min. at 37° C. The mixture is brought to 10mM EDTA, extracted 2 x phenol-$CHCl_3$, 1 x ether and desalted by centrifugation through 0.5ml Sephadex G-25 equilibrated with water.

A major portion of the resulting circular ssDNA is combined with 50pmole of the 5'-phosphorylated oligonucleotide, depicted above for introducing the PstI site, in 38μl of 200mM NaCl, 13mM Tris-HCl, pH 7.5, 9mM magnesium acetate, 20mM β-mercaptoethanol, boiled for 30min and immediately cooled to 0° C. After adding 5 μl of a solution 4mM in the four dNTPs, 0.5 μl of 100mM ATP, 3μl (3 units) of DNA polymerase I (Klenow fragment) and 4μl (10 units) of T4 DNA ligase, the mixture is incubated overnight at 12° C and then used directly for transformation of E. coli HB101; the transformants are grown, isolated and analyzed using blot hybridization employing radiolabeled $^{32}P$-oligomer to detect clones having the tailored sequence containing the new PstI site.

The "tailored" pGMI is isolated and partially restricted with PstI, and the DNA sequences coding for the light and heavy chain variable regions prepared above are inserted individually into the tailored site to provide two plasmids having DNA sequences coding for the light (pGMIL) and heavy (pGMIH) chains, in accordance with the procedure described previously for insertion. The resulting plasmids are used to transform E. coli HB 101, and clones having the light and heavy variable region sequences in the desired orientation are identified by restiction mapping and purified.

Antisera recognizing the light and heavy chains respectively are produced by using the particular chains as immunogens, and the antisera isolated and covalently linked to Sepharose by conventional procedures (March et al., Anal. Biochem. (1974) 60:149-152) and the products employed for affinity columns.

The transformants are grown to cell densities of about $10^9$ cells/ml and collected by centrifugation. The pellet is resuspended in 50 μl of 50mM Tris-HCl, pH8, 50mM EDTA, 15% sucrose, 1mg/ml lyzosyme, 0.5% NP40. After 30min at 0° C, 10μl of 150mM Tris-HCl, pH 7.5, 280mM $MgCl_2$, 4mM $CaCl_2$ and lμg DNase are added, followed by centrifugation for 15min at 12,000g.

The protein is then isolated by removal of the supernatant from the pellet and the supernatants are passed over the immunosorbent columns (0.15ml) equilibrated with Tris-HCl, pH 7.5. The light and heavy chains of the rFv are eluted with 1M acetic acid, pH 2.5 and the eluates pooled and neutralized with 0.1M NaOH at 0° C to pH 5.5. The pooled eluates are dialyzed against 3 x 100 volumes of sodium acetate buffer, pH 5.5, followed by 3 x 100 volumes PBS, pH 7.

If the renatured light and heavy chains of the rFv have the same source, they can be further purified by combining the eluates containing the rFv components and passing them over a DNP-affinity column. (In the present example, different sources of heavy and light chains are described, so that this seep is omitted.) A DNP-affinity column and procedure are described by Kooistra and Richards, Biochem. (1978) 17:345-351. The bound rFv is isolated by elution with 1M acetic acid, followed by renaturing with sequential dialysis as described above. In addition, sulfhydryl groups may be capped with iodoacetamide as described by Kooistra and Richards, ibid.

The present method provides protein complexes of homogeneous composition having two peptide chains which form a complex having high binding affinity for a predetermined haptenic site. The two chains form an rFv having specificity for a particular ligand, by mimicking a naturally occurring immunoglobulin. Without the constant regions, the resulting rFv has reduced immunogenicity and lacks peptide sequences which may have undesirable functions for particular applications e.g. complement fixation.

The rFv can be used for a variety of purposes and diagnosis and therapy. The composition is homogeneous and therefore has a fixed reproducible level of immunogenicity. Also, owing to the reduced molecular weight, it will have relatively short residence times after injection into a mammalian host. This is particularly important where the rFv is labeled for diagnosis or therapy employing hazardous labels, such as radionuclides, heavy metals, cytotoxic agents, and the like. Short residence times can also be important where the rFv is used to inhibit physiologically active materials in vivo e.g. hormones, enzymes, surface receptors, lymphocytes or other cells, and the like.

The uniform composition allows for controlled labeling, enhancing the ability of a conjugate to label a particular site on one or the other or both of the chains. The uniformity permits controlled conjugations, accurate determinations of therapeutic activity, easy monitoring of therapeutic effect, enhanced reproducibility of results and control and ease of monitoring of side effects.

The present invention provides for accurate synthesis of polypeptide chains which can be brought together to form a binding site for a predetermined epitopic site. The light and heavy chains prepared by the present method can be brought together in the presence or absence of the ligand. Also, the invention permits introduction of a particular amino acid at either terminus of a chain for particular applications, e.g. tyrosine for radioiodination. By using monoclonal hybridomas as the source of the DNA for coding the variable regions, the naturally occurring binding efficiency is retained and binding affinity can be widely varied.

## Claims

1. A specific binding composition comprising two polypeptide chains of an immunoglobulin having binding specificity to a predetermined ligand, characterized in that said two polypeptide chains
   (1) have the amino acid sequence of at least a portion of the variable region of an immunoglobulin, but substantially lack the constant region,
   (2) associate to form a complex having a high affinity and specificity for said predetermined ligand, and
   (3) are the light chain of from about 95 to 115 amino acids and the heavy chain of from about 110 to 125 amino acids, wherein said heavy chain includes the D-region.

2. A composition as claimed in claim 1 wherein each of said chains is labelled with a functionality capable of producing a detectable signal.

3. A composition as claimed in claim 2, wherein said functionality is a cytotoxic agent or a radionuclide.

## Revendications

1. Composition spécifique de liaison comprenant deux chaînes polypeptidiques d'une immunoglobuline ayant une spécificité de liaison pour un ligand prédéterminé, caractérisée en ce que lesdites deux chaînes polypeptidiques
   (1) ont la séquence d'acides aminés d'au moins une portion de la région variable d'une immunoglobuline, mais manquent sensiblement de la région constante.
   (2) s'associent pour former un complexe ayant une forte affinité et une forte spécificité pour ledit ligand prédéterminé, et
   (3) sont la chaîne légère d'environ 95 à 115 acides aminés et la chaîne lourde d'environ 110 à 125 acides aminés, où ladite chaîne lourde comprend la région D.

2. Composition selon la revendication 1, où chacune desdites chaînes est marquée d'une fonctionnalité capable de produire un signal détectable.

3. Composition selon la revendication 2, où ladite fonctionnalité est un agent cytotoxique ou un radionuclide.

## Ansprüche

1. Spezifische Bindungszusammensetzung, umfassend zwei Polypeptid-Ketten eines Immunglobulins mit Bindungsspezifität gegenüber einem vorbestimmten Liganden, dadurch gekennzeichnet, daß die zwei Polypeptid-Ketten
   (1) die Aminosäuresequenz wenigstens eines Teils der variablen Region eines Immunglobulins aufweisen, ihnen aber im wesentlichen die konstante Region fehlt,
   (2) assoziieren unter Bildung eines Komplexes, der hohe Affinität und Spezifität gegenüber dem vorbestimmten Liganden aufweist, und
   (3) die leichte Kette von etwa 95 bis 115 Aminosäuren und die schwere Kette von etwa 110 bis 125 Aminosäuren darstellen, wobei die schwere Kette die D-Region einschließt.

2. Zusammensetzung nach Anspruch 1, wobei eine jede Kette mit einer Funktionalität versehen ist, die ein meßbares Signal erzeugen kann.

3. Zusammensetzung nach Anspruch 1, wobei die Funktionalität ein cytotoxisches Agens oder ein Radionuklid ist.